# EUROPEAN PATENT APPLICATION

(11) **EP 0 839 524 A1**
(43) Date of publication of application: **06.05.1998**
(21) Application number: 97118402.3
(22) Date of filing: 23.10.1997
(51) Int. Cl.: A61K 9/00, A61K 31/44, A61K 31/47, A61K 47/32

(54) **Pharmaceutical compositions for the oral use containing disinfectant substances and muco-adhesive substances**

(30) Priority: 29.10.1996 IT MI962239
(71) Applicant: Montefarmaco S.p.A., 20121 Milano (IT)
(72) Inventor: Ronchi, Celestino, 20016 Pero (MI) (IT); Berlati, Fabio, 20016 Pero (MI) (IT)
(74) Representative: Spadaro, Marco

(57) **Abstract**

Solid oral pharmaceutical compositions in the form of lozenges comprising at least an active ingredient in admixture with conventional carriers and excipients, characterized in that they contain at least one muco-adhesive polymer.

## Description

### TECHNOLOGICAL BACKGROUND

The administration of disinfectant substances (iodine, ammonium quaternary salts, such as cetylpyridinium chloride, dequalinium chloride, benzethonium chloride, or others such as dichlorobenzyl alcohol, amyl metacresol, silver proteinate, methylisoprinol) or of real antibiotic substances with topical action (such as tyrothricin or fusafungine) has been used for a long time in the treatment of some conditions of infective origin (bacterial or fungine) typical of the pharyngo-oral cavity and of the periodontal areas.

The topical administration of the various active ingredients is performed by means of the pharmaceutical forms conventionally employed for the topical use in the oropharynx, such as tablets, collutories, tinctures, sugar or no-sugar lozenges (containing xylitol, sorbitol or other non-cariogenic polyalcohols).

This type of therapy, although being widespread and having good compliance, is known to have limited efficacy.

Among the main factors limiting the efficacy of the topical therapy of the infections of the oral cavity, the poor contact time between the oral mucosa and the active ingredients and the presence of effective concentrations of active ingredients for rather short times should be stressed; said effects being mainly due to the dilution and washing away by the saliva.

A method to improve the permanence of the active ingredients at the action site, in order to increase the efficacy of the medicament, is preparing pharmaceutical forms which make use of the properties of polymer muco-adhesive substances. Muco-adhesion has been studied extensively in the eighties and applied in the development of pharmaceutical forms for the purpose of improving the absorption of the active ingredients.

Among the muco-adhesive substances, cellulose derivatives (cellulose derivatives, such as the methyl, hydroxypropylmethyl, carboxymethyl ethers or esters, etc.), polyacrylic acid derivatives (polycarbophyl, such as Noveon^{(R)}); vinylcarboxyl acid derivatives (carbomer, such as Carbopol^{(R)} 9749), should be mentioned.

At present, muco-adhesive pharmaceutical forms are developed above all as forms for carrying and absorbing substances difficult to absorb, such as peptides. The muco-adhesive has up to now been used to bind the pharmaceutical form, usually consisting of an insoluble or slowly erodible matrix, to the mucosa (buccal, gastric, nasal) designed to absorb the medicament. Thus, the muco-adhesive substances have been administered both as constituents of a separate adhesive layer and as constituents of the matrix itself.

Up to now, the development of crystal lozenges containing muco-adhesive substances has not been studied or used in practice.

The sugar- or no-sugar- lozenges are pharmaceutical forms in which the main ingredient is a sugar (saccharose) or a polyalcohol such as sorbitol or xylitol. The preparation technique used is a conventional technique involving melting and "cooking" the main mass and the subsequent subdivision either by pouring the flavoured mass, enriched in the active ingredient and in the muco-adhesive, into starch moulds or drawing dies or by extrusion and subsequent cutting of the extruded mass. The divided mass is cooled and left to crystallize.

Up to now, the administration of the bioadhesive polymers in sugar- or no-sugar lozenges has not been described, due to the difficulty in dispersing homogeneously in a high temperature melted mass a biopolymer in minimum amounts (0.1 - 1.0%) compared with the final mass of the lozenge (which has usually a final weight higher than 2 - 3 g) and in causing no structural distortions of the final product due to the permanent set of the biopolymer caused by the excessive temperature.

Now it has been found that the dispersion of the polymer has to occur after decreasing the temperature of the melted base mass to a critical range and mixing in the melted mass itself a homogeneous phase resulting from admixture of the excipients, present in small amounts (even if non aqueous liquids, such as essential oils or flavours, or pasty substances) with the muco-adhesive and optionally with the active ingredient when it is present in minimum amounts or is thermolabile.

The present invention relates to lozenges based on sugar or no-sugar excipients, obtained with the technology described herein, containing active ingredients with topical action for use in the pharyngo-oral cavity in combination with anhydrous muco-adhesive substances which provide, once hydrated, a longer-lasting action and a higher efficacy.

A further object of the present invention is a process for the preparation of the lozenges cited above.

### Detailed disclosure of the invention

The present invention relates to the use of a muco-adhesive substance for the preparation of lozenges which, when dissolved in the mouth, provide the simultaneous release of both the active ingredient and the muco-adhesive substance, which hydrating with the saliva makes the active ingredient to adhere to the pharyngo-oral mucosa. More precisely, the lozenges contain disinfectant active ingredients which are intended to exert their action topically, at the mucosa surface, instead of being absorbed.

Therefore the type of administration provides lozenges for the pharmaceutical use in which the adhesiveness of the biopolymer, deriving from the hydration due to the contact with saliva, keeps the active ingredient in contact with the oral mucosa for a protracted time, thus making its action longer-lasting.

The pharmaceutical compositions according to the present invention comprise one or more active ingredients in admixture with conventional carriers and excipients and one or more muco-adhesive polymers.

The muco-adhesive polymers used in the present invention are known to those skilled in the art.

Among these, the following ones are cited by way of example: polycarbophil, polyacrylic acid derivatives and the salts thereof, vinylcarboxylic acid polymers and copolymers and the salts thereof, cellulose derivatives such as methyl, ethyl, hydroxyethyl, propyl, hydroxypropyl ethers or esters methyl, carboxymethyl ethers and the like.

The bioadhesive polymers can be in a combination thereof and are used in percentages of 0.1% to 50% by weight compared with the total composition. The pharmaceutically acceptable carrier is a substance or a mixture of substances in which the bioadhesive polymer(s) is(are) dispersed or dissolved and which can be administered to humans or other animals without causing adverse effects, such as toxic effects or irritations. The components of the carrier are powders, liquids, pasty substances which are usually employed in pharmaceutical, alimentary and dietetic preparations.

The composition consists of excipients in a percentage ranging from 30% to 99.9%.

The active ingredients present in the formulations are of various nature (natural, synthetic, semisynthetic) and are characterized by different therapeutical and prophylactic uses in the various pathologies of the pharyngo-oral cavity and of the periodontium (antibacterials, antimycotic and topical antiseptics) and they are present in the pharmaceutical form in concentrations of 0.001% to 50%.

Examples of the active ingredients used in the present invention are disinfectants (iodine, quaternary ammonium salts, such as cetylpiridinium chloride, dequalinium chloride, benzethonium chloride, or other such as dichlorobenzyl alcohol, amyl metacresol, silver proteinate, methylisoprinol) or real antibiotic substances with a topical action, such as tyrothricin or fusafungine.

The pharmaceutical compositions according to the present invention are in the form of crystal lozenges containing sugar (saccharose, fructose, or other crystallizable sugars) or without sugar, based on polyalcohols (sorbitol, xylitol, mannitol, isomaltol, maltitol and the like).

According to the present invention, the process for preparation of said lozenges comprises the following steps:
a) preparing a melted base mass for lozenges according to conventional methods;
b) lowering the temperature of the melted base mass to a range from 45° to 75°C;
c) adding the muco-adhesive, optionally in admixture with other excipients, in conditions of strong stirring until homogeneous distribution;
d) preparing the single dosage forms.

Lower temperatures prevent a complete homogenization (in fact the mass is already in the crystallisation phase) whereas higher temperatures cause structural permanent sets of the bioadhesive polymer which change the shape and technological characteristics of the product.

The active ingredient, depending on its the amount and nature, can be added directly to the base mass to melt, or it can be pre-mixed with the muco-adhesive and optionally other excipients, then added to the melted mass.

Excipients and carriers for the compositions according to the present invention are known in the art, for example as described in "Remington's Pharmaceuticals Sciences Handbook", Mack. Pub., N.Y., U.S.A..

In an embodiment of the invention, in the preparation of the lozenges as described in example 1, the muco-adhesive polycarbophil (Noveon^{(R)} AA1) is dispersed in anethole, menthol, eucalyptol, pumilius pine essential oil and peppermint oil and mixed until obtaining a homogeneous pasty mass which is subsequently distributed, under strong stirring for at least for 10 minutes, into the melted mass kept at a temperature ranging from 45° to 75°C.

The compositions of the invention evidenced that, the active ingredient dosages being equal, a topical effective concentration of medicament can be maintained for a time substantially more prolonged than with the conventional pharmaceutical forms, thereby decreasing the administrations and allowing a shorter treatment time.

The following examples further illustrate the invention:

### Example 1

Disinfectant sugar lozenges containing a quaternary ammonium salt (Dequalinium Chloride).

| | |
|---|---|
| Dequalinium Chloride | 0.25 mg |
| Polycarbophil (Noveon^{(R)} AA1) | 12.50 mg |
| Saccharose | 1580 mg |
| Glucose | 1400 mg |
| Menthol | 12 mg |
| Eucalyptol | 3 mg |
| Anethole | 1.5 mg |
| Pumilius pine essential oil | 1.5 mg |
| Peppermint oil | 0.9 mg. |

### Example 2

Disinfectant "no-sugar" lozenges containing a quaternary ammonium salt (Dequalinium Chloride).

| | |
|---|---|
| Dequalinium Chloride | 0.25 mg |
| Polycarbophil (Noveon^{(R)} AA1) | 12.50 mg |
| Sorbitol | q.s. to 2.5 g |
| Vegetable soft extracts | 25.00 mg |
| Peppermint oil | 5.00 mg |

### Example 3

Disinfectant "no-sugar" lozenges containing a quaternary ammonium salt (Cetylpiridinium Chloride).

| | |
|---|---|
| Cetylpiridinium Chloride | 1.20 mg |
| Polycarbophil (Noveon^{(R)} AA1) | 12.50 mg |
| Sorbitol | q.s. to 2.5 g |
| Vegetable soft extracts | 25.00 mg |
| Peppermint oil | 5.00 mg. |

## Claims

1. Solid oral pharmaceutical compositions in the form of lozenges comprising at least one active ingredient in admixture with conventional carriers and excipients, characterized in that they contain at least one muco-adhesive polymer.

2. Compositions according to claim 1 wherein the muco-adhesive polymer is contained in amounts ranging from 0.1 to 50% by weight.

3. Compositions according to claim 1 or 2, wherein the muco-adhesive polymer is selected from the group consisting of: polycarbophil, polyacrylic acid derivatives and the salts thereof, cellulose derivatives, carboxymethyl ethers, vinylcarboxylic acid derivatives.

4. Compositions according to any one of claims 1 to 3, containing 30 to 99.9% by weight of excipients.

5. Compositions according to any one of claims 1 to 4, wherein the active ingredients are contained in amounts ranging from 0.001 to 50% by weight.

6. Compositions according to claim 5, wherein the active ingredients are selected from the group consisting of antibacterials, antimycotics and antiseptics.

7. Compositions according to claim 6, wherein the active ingredients are selected from the group consisting of: iodine, quaternary ammonium, cetylpirinium chloride, dequalinium chloride, benzethonium chloride, dichlorobenzyl alcohol, amyl metacresol, silver proteinate, methylisoprinol, tyrothricin, fusafungine.

8. Compositions according to any one of claims 1 to 7, containing non-cariogenic sugars.

9. Compositions according to claim 8, containing one non-cariogenic sugar selected from the group consisting of sorbitol and xylitol.

10. A process for the preparation of the compositions of claims 1 to 9, which comprises the following steps:
a) preparing a melted base mass for lozenges according to conventional methods;
b) lowering the temperature of the melted base mass to a range from 45° to 75°C;
c) adding the muco-adhesive, optionally in admixture with other excipients, in conditions of strong stirring until homogeneous distribution;
d) preparing the single dosage forms.

11. A process according to claim 10, wherein the active ingredient is added directly to the base mass, or is pre-mixed with the muco-adhesive and optionally other excipients, then added to the melted mass.
